# EUROPEAN PATENT APPLICATION

(11) **EP 3 763 828 A1**
(43) Date of publication of application: **13.01.2021**
(21) Application number: 19184933.0
(22) Date of filing: 08.07.2019
(51) Int. Cl.: C12Q 1/689

(54) **METHOD FOR MONITORING FERMENTATION PROCESSES, APPARATUS, AND SYSTEM THEREFORE**

(71) Applicant: Nemri, Adnane, 37079 Göttingen (DE); Kumar Patel, Vipul, 34090 Montpellier (FR); Limes Innovations B.V., 2351 SX Leiderd (NL)
(72) Inventor: Nemri, Dr. Adnane, 37079 Göttingen (DE); Kumar Patel, Dr. Vipul, 34090 Montpellier (FR)
(74) Representative: Gramm, Lins & Partner Patent- und Rechtsanwälte PartGmbB

(57) **Abstract**

The present invention relates to a method for monitoring fermentation processes as well as a method for predicting fermentation processes and a method for regulation fermentation processes. Further, computer program for running the method according to the present invention as well as apparatus comprising the same and a system for at least monitoring, predicting or regulating the fermentation process according to the present invention is provided.

## Description

In a first aspect, the present invention relates to a method for monitoring fermentation processes as well as a method for predicting fermentation processes and a method for regulating fermentation processes. Further, computer program for running the method according to the present invention as well as apparatus comprising the same and a system for at least monitoring, predicting or regulating the fermentation process according to the present invention is provided.

### Prior art

Fermentation is a process in which organic matter is decomposed in the absence of oxygen producing an accumulation of resulting fermentation products. Fermentation is primarily an anaerobic process converting sugars, such as glucose, to other compounds like alcohol producing energy for the microorganism or cell. Fermentation has been used by humans for thousands of years, with major roles in food preservation and alcohol production. Some of these fermentation products have a high economical value due to their contribution to human or animal nutrition and health and their use in industrial application.

Microorganisms with the enzymatic capacity for fermentation include bacteria, e.g. conducting lactic acid fermentation, and fungi, like yeast which perform ethanol fermentation. Many different products are a result of fermentation, either occurring due to the naturally attached microorganisms or through the addition of a starter culture.

During lactic acid fermentation, the pyruvate molecules from glycolysis are converted into lactate. Lactic acid bacteria (LAB) consist of homo and hetero-lactic acid organisms, and represent a broad range of bacteria, including *Lactobacillus, Streptococcus, Enterococcus, Lactococcus* and *Bifidobacterium,* with the ability to produce lactate primarily from sugars. They are among the most commercially used bacteria today contributing to yogurt, sauerkraut, kimchi and kefir production as well as pickling of vegetables, curing of fish, and many other traditional dishes around the world.

To the actions of various yeasts as an example for fungi, alcoholic fermentation produces carbon dioxide and ethanol from pyruvate molecules as an example for alcoholic fermentation. *Saccharomyces cerevisiae* is used in bread making, helping the dough rise through the production of carbon dioxide, other strains of *S*. *cerevisiae* are used e.g. in alcohol production including beer and wine in combination with other yeast species.

Another example is the production of fermented crops, so called silage, for animal feed. The quality of silage has a relevant impact on animal welfare and performance measures including milk production in dairy cattle.

Fermentative bacteria and yeasts are referred to as "probiotic" when they adhere to the following WHO definition "live microorganisms which, when administered in adequate amounts, confer a health benefit on the host".

During silage production, several fermentative organisms can be added for producing vitamins, improving the quality and, thus, the value of the food. The fermentation products in silage have also a conservation effect making it an attractive alternative feed during wintertime, when green food is not available. Fermented silage can be used for biogas production and energy production as well. However, during fermentation process problems can occur which can have a negative effect on the quality of the end product, which may be through the presence of toxic or undesirable byproducts, causing the product to not pass regulations and rendering it unusable, leading to capital loss. For example, toxic byproducts including mycotoxins, ethyl carbamate, medium chain fatty acids, acetic acid, acetaldehyde, or pathogen appearances including *Bacillus cereus, Salmonella spp., Escherichia coli O157:H7, Staphylococcus aureus, Vibrio cholera, Listeria monocytogenes, Aeromonas, Klebsiella, Campylobacter and Shigella sp.* are the results of fungal or bacterial contaminations and/or unfavorable environment conditions during the process of fermentation.

The premature end of fermentation can have multiple reasons, for example not having adequate levels of essential component necessary for fermentation including nitrogen, amino acids, sterols, fatty acids, vitamins, minerals, the environmental conditions including humidity, pH, sugar, uncontrolled temperature or adequate microbial community, thus, leading to oxidation or prevalence of undesirable microorganisms in the fermented product. For silage fermentation there are some known problems, i.e. clostridia contamination which produce butyric acid resulting in protein degradation, reduction of palatability and energy reduction. High aerobic conditions from poor packing or low moisture levels can have an effect on the total dry mass lost as well as on the palatability and digestibility. One further problem is the production of ethanol in silage by spoilage yeast mixed with acetic acid resulting in lower feed quality.

Individuals performing fermentation reactions, such as farmer and technical engineers in case of biogas facilities making silage or a homecook making pickled vegetables, typically are faced with limitations compared to industrial producers of fermented products, such as large-scale yoghurt manufacturers. Such limitations include relatively large batch sizes and vast numbers of uncontrolled variables resulting in variations in end-product quality and consistency. Being limited to one or a few batches with potentially large stakes also limits the ability of users to experiment and modify aspects of the fermentation process to improve the amount or quality of fermented product obtained.

For example, fermented products can obtain microbes and different compounds resulting from microbial activity, e.g. vitamins or toxins. Estimating the health impact of ingesting fermented product is noticeably difficult, as it requires first estimating the presence and abundance of different microbes and/or their by-products, and then estimating how these microbes or their by-products affect health aspects. For example, it is not clearly established how different abundance of mycotoxin-producing fungi in silage is negatively affecting milk production and general health in dairy animals.

It is considered that a vast amount of living microorganisms is not yet catalogued and cannot be identified by matching DNA sequences against databases of known microbial species, i.e. by applying the common 16S or ITS approach. As a result, metagenomics sequencing of environmental samples typically produces a large fraction of unidentified reads, that are not usable in subsequent analysis.

Current strategies to counteract the above-mentioned scenario include the use of additives, either biological or chemical, on the silage or the other fermentation products. These additives can be collections of bacteria or chemical compounds. However, as mentioned, various obstacles are given during the fermentation process and the outcome is difficult to predict.

### Brief description of the present invention

The processes described herein are useful for improving fermentation processes with respect to the quality of their outcome. The advantages of the processes according to the present invention are demonstrated for the production of silage, the process whereby fermentation changes whole plant material into a durable and healthier feed. Typically, silage is produced by an anaerobic fermentation, starting already after 48 hours after a silo is filled and converting the sugar content into acid. The whole process takes only two weeks. There are multiple factors which control the quality outcome of the fermentation process such as the level of moisture, oxygen level, temperature, sugar content and of course, the metagenomics composition of the silo. The level of acidity after fermentation can improve the palatability and provides a dietary benefit for the animal, but excessive acidity can reduce drastically palatability. Hence, controlling the silage during the fermentation is a crucial step.

According to the present invention, the process is a method for monitoring fermentation processes comprising the steps of
a) obtaining a sample from the fermentation process;
b) determining a pattern including metagenomic marker;
c) comparing the pattern including metagenomic marker with pattern comprising metagenomic marker from a database;
d) correlating the comparison in step c) with the ongoing fermentation process and predicting the fermentation process outcome.

In another aspect, the present invention provides a method for predicting fermentation processes
a) obtaining a sample from the fermentation process;
b) determining a pattern comprising metagenomic marker,
c) comparing the pattern comprising metagenomic marker with pattern comprising metagenomic marker from a database;
d) predicting the further fermentation process, in particular, the outcome of the fermentation process based on the comparison in step c).

In a further aspect, the present invention provides a method for regulating fermentation process
a) obtaining a sample from the fermentation process;
b) determining a pattern comprising metagenomic marker;
c) comparing the pattern comprising metagenomic marker with pattern comprising metagenomic marker from a database;
d) regulating the ongoing fermentation process based on the comparison in step c).

In another aspect, a computer program product residing on a computer readable medium, with a program code when it is running on a computer or is loaded on a computer, causes the computer to perform the method according to the present invention is provided. Further, an apparatus comprising the computer readable storage medium containing program instructions for conducting the method according to the present invention when it's running on a computer and a system for at least one of the methods provided comprising a device for obtaining a pattern comprising metagenomics marker and a data processing unit comprising a software or a computer program product according to the present invention for carrying out the method according to the present invention.

### Brief description of the drawings

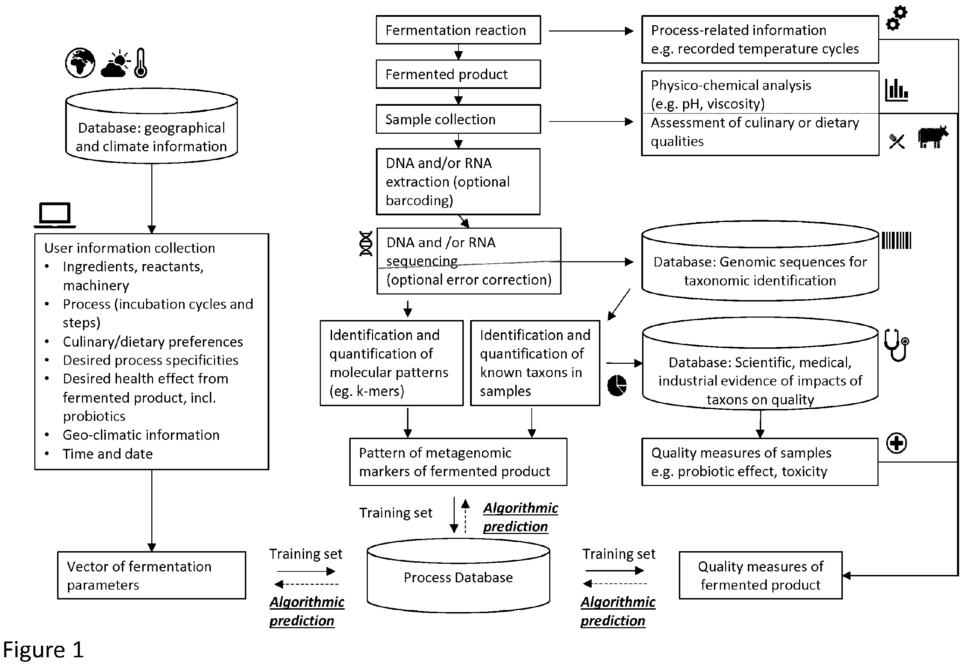

Figure 1 is a flowchart of the methods according to the present invention.

### Detailed description of the present invention

In a first aspect, the present inventions related to a method for monitoring fermentation processes comprising the steps of
a) obtaining a sample from the fermentation process;
b) determining a pattern including metagenomic marker;
c) comparing the pattern including metagenomic marker with pattern comprising metagenomic marker from a database;
d) correlating the comparison in step c) with the ongoing fermentation process and predicting the fermentation process outcome.

In a further aspect, the present invention relates to a method for predicting fermentation processes comprising the steps of
a) obtaining a sample from the fermentation process;
b) determining a pattern comprising metagenomic marker,
c) comparing the pattern comprising metagenomic marker with pattern comprising metagenomic marker from a database;
d) predicting the further fermentation process, in particular, the outcome of the fermentation process based on the comparison in step c).

Moreover, the present invention provides a method for regulating fermentation processes comprising the steps of
a) obtaining a sample from the fermentation process;
b) determining a pattern comprising metagenomic marker;
c) comparing the pattern comprising metagenomic marker with pattern comprising metagenomic marker from a database;
d) regulating the ongoing fermentation process based on the comparison in step c).

That is, the present invention relates to novel and unique methods and processes for monitoring and controlling natural fermentation processes. Fermentation activities entail a wide range of endeavors directed toward agriculture, manufacturing and chemical processing.

The current disclosure relates to the field of biological fermentations, in particular, to the field of food and feed technology. More particular, the disclosure concerns inventive methodology that may be useful in improving the quality of fermentation products. In particular, the invention may be useful in reducing contaminants and in improving food properties related to animal and human health. The processes described herein are useful for improving any fermentation process with respect to the quality of its outcome. The advantages of the processes according to the present invention are shown for the production of silage, the process whereby fermentation changes whole plant material into durable and healthier feed.

The present invention purposes to mitigate the problem known in the art by allowing individual users to harvest the wealth of information gathered by other individual users present in a database. The invention pools process information from multiple sources to enable uses to simulate and predict fermentation reaction outcomes via computational algorithms including machine learning or deep learning. For example, the algorithm predicts for users whether their input parameters in conjunction with their geographical location and their local climatic conditions would be likely into result in their desired outcomes, based on instances of users in close geographical range with similar climatic conditions and fermentation inputs. That is, the method according to present invention uses a collection of data present in a database gathered from fermentation processes including the reaction parameters and reaction outcomes during one or several time points. That is, according to the method for monitoring fermentation processes, for predicting fermentation processes and/or for regulating fermentation processes includes the step of determining a pattern comprising metagenomic marker.

The herein described process includes systems and methods for determining and characterizing the metagenome and microbiome of a fermentation operation or setting, predicting the fermentation outcome based on data collection at least based on DNA/RNA patterns of the metagenomic analysis and suggesting parameters to improve the outcome of the fermentation. The methods according to the present invention allows a real time snapshot, monitoring of the current fermentation status.

The term "metagenomic marker" as used herein, refer to genetic material based marker. Metagenomic marker include DNA or RNA read sequences that can be assigned to a likely taxon of origin or k-mers derived from those DNA or RNA reads.

The term "k-mer" refers to all the possible subsequences of the length k from a read obtained through DNA sequences. The amount of k-mers is possible given a string of length L is L-k+1, whilst the number of possible k-mer is given n possibilities (4 in the case of DNA, namely ACTG) is n^{k}. For example, the k-mers are determined as molecular pattern from metagenomic sequencing as suitable metagenomic marker representing variables in metagenomic prediction and associate the pattern including the metagenomic marker with specific fermentation parameters and/or fermentation outcomes including health impact from guessing the fermented products.

That is, the generation of k-mers allows to create pattern or signature of genetic material. The terms "pattern" and "signature" are used herein interchangeably. Comparing the frequencies are computationally easier than sequenced alignment, thus, allowing alignment free sequence analyses.

That is, in an embodiment of the present invention, the methods according to the present invention for monitoring, predicting and/or regulating fermentation processes is a process wherein the metagenomic marker include DNA/RNA pattern. In an embodiment, the metagenomic data include data on the microbiome present in said sample.

For example, the metagenomic marker include the k-mer pattern or signature from metagenomic sequencing. The microbiome present in the sample obtained from the fermentation process represents a variable tool for monitoring the fermentation process for predicting the fermentation process and for regulating the fermentation process. This is particularly true when comparing the data from the fermentation process with data gathered from fermentation processes present e.g. in a database obtained at several time points. The metagenomic pattern or signature may be connected with other parameters of the fermentation processes including time, place or temperature and control variables, such as ingredients and machinery. Further, physico-chemical parameters can be included.

In an embodiment, the fermentation process in the methods according to the present invention is a biological fermentation process.

In ananother embodiment, the methods according to the present invention the determination of the pattern comprising metagenomic marker is a quantification of said metagenomic marker. The quantification include a semi-quantitative determination of said metagenomic marker. The skilled person is well aware of suitable methods for obtaining the metagenomic marker including the k-mer.

The database for use in the method according to the present invention contain data on pattern comprising metagenomic marker from fermentation processes. Typically, these metagenomic markers, present in the database, are linked with other fermentation parameters and/or fermentation outcomes. Hence, in an embodiment, the present invention relates to methods according to the present invention further comprising determining additional metainformation before or at the time point when obtaining the sample from the fermentation process and comparing pattern comprising metagenomic marker and metainformation of said sample with the pattern comprising metagenomic marker and metainformation from a database.

The term "metainformation" refers to any variable which may not be controllable or without clear or previously known effect on the fermentation product. The term "metainformation" relate *inter alia* to date or time of the fermentation process and geolocation of the fermentation process as well as past, current and predicted future weather information for the specific location of said fermentation process.

These metainformation are part of the database.

That is, in an embodiment of the present invention, the metainformation obtained for the sample before or at the time point when obtaining the sample from the fermentation process according to the present invention include date or time and geolocation of the fermentation process as well as past, current and predicted future weather information for the specific location of said fermentation process.

In a further embodiment, the method according to the present invention is a method including determining reaction parameters of the fermentation reaction obtained before or at the time point when obtaining a sample from the fermentation process according to step a), comparing said reaction parameters in combination with the pattern comprising metagenomic marker, and, optionally, metainformation with reaction parameters in combination with the pattern comprising metagenomic markers and, optionally, metainformation, from a database to predict or regulate the future fermentation process. The reaction parameters include batch number, operators, ingredients and reactants, ferments, machinery, their quantities and/or properties; process related information including recorded temperature cycles; physico- chemical analysis including pH, viscosity, assessment of culinary/dietary qualities using which further data may be retrieved from database such as chemical composition of an intrant, brand identification of an intrant derived from a description or a barcode, predicted health impact of fermented product used.

In an embodiment, the method according to the present invention wherein prediction include the prediction of quality of the fermentation product and/or the future pattern of metagenomics marker of the fermented product. That is, the methods according to the present invention allows monitoring of the fermentation process with the goal of predicting the fermentation process outcome. In particular, the method is a method regulating the ingrowing fermentation process based on the comparison of at least the metagenomics marker with pattern comprising said metagenomics marker from a database. Optionally, in combination with at least one of the additional meta-information before or at the time point when obtaining the sample from the fermentation process or reaction parameters of the fermentation reaction. Suitable meta-information parameters and reaction parameters are shown in the flowchart of figure 1.

In a further embodiment of the present invention, the method is a method further comprising corrective interventions to improve the future quality of the fermented product based on the known impacts of interventions on quality of fermented products from a database.

A further aspect relates to a method according to the present invention wherein the fermentation process is a fermentation process for the production of food or feed.

In this connection, the term "outcomes" or "outcome" include the characterization of microbial communities from fermented foods or feeds and/or physico-chemical measurements of those fermented foods and feeds. A feature vector capturing all recorded parameters and outcomes will be constructed from which a machine-learning or deep-learning algorithm can select a subset of the reaction parameters and/or outcomes to predict a subset of other reaction parameters and/or outcomes. Examples of such predictions include:
- Given certain initial reaction parameters, a prediction of physio-chemical properties of the fermented food or feed
- Given certain initial reaction parameters, a prediction of compositions of the microbial communities in the fermented food or feed
- Given signal distributions derived from sequencing of the microbial communities, a prediction of physico-chemical properties of the fermentation process
- Given reaction and signal parameters, a prediction of the quality of the fermentation process for the applied food or feed
- From reaction outcomes such as signals derived from sequencing the microbial communities, a prediction of the health impact of the fermented food or feed on the target consumers
- Given a fermentation outcome, a prediction for certain initial reaction parameters
- Given a desired fermentation outcome for a given time point given observed reaction parameters as prediction how to modify to a possible optimum.

The present invention, by measuring microbial presence combined with the abundance in collecting performance related indicators of health from individuals from individual uses can achieve sufficient power to predict whether a fermented product is likely to contribute negatively or positively to health and/or performance. For example, the microbial profile of a silage feed can be used to predict the performance of lactating cows of a specific breed based on data collected for similar microbial profiles. Information can be deduced as to the suitability of this feed and the need for a corrective measure to restore animal performance.

Mainly, resolving the microbiome of fermented products and how it changes with time would be advantageous and necessary for influencing the quality of fermented products. By combining different technologies and concept which have been herein described, it is now possible to generate a real time overall profile of the fermentation process, based on its microbiome and other sensor-data. Such profiles would allow producers to predict silage quality already in the early stages and solve the described problems using individually designed solutions which is in contrast to current methods where a predefined mix of microbes or chemicals is supplied. Interventions can be performed throughout the course of the fermentation though changes of physical chemical parameters including temperature, pH, enzymes or adding certain organisms in the process in influencing the microbial community.

In an embodiment of the invention, the comparing step c) and the correlating, predicting or regulating step d) is effected by software.

In another aspect, the present invention relates to a computer program product residing on a computer-readable medium, with a program code when it is running on a computer or is loaded on a computer, causes the computer to perform a method according to the present invention. In an embodiment, a computer program storage medium which comprises the computer program product according to the present invention is provided.

The person skilled in the art is well aware of suitable storage media accordingly.

In a further aspect, an apparatus comprising a computer-readable storage medium containing program instructions for carrying out the method according to the present invention when its running on a computer is provided. Further, a system for at least one of monitoring a fermentation process, predicting a fermentation process or regulating a fermentation process for carrying out the method according to the present invention comprising a device for obtaining a pattern comprising metagenomics marker and a data processing unit comprising a software or a computer program according to the present invention is provided. The skilled person is well aware of suitable systems including input and output units etc.

The present invention will be described further by way of referring to the flowchart of figure 1. Namely, the forgoing and other features and advantages of the present invention will be apparent from the following more particular description of various exemplary embodiments as illustrated in the flowchart of claim 1 and referring to the different process steps.

Figure 1 is a flowchart showing the various aspects of the methods according to the present invention. Namely, in the middle of this flowchart, the fermentation process is identified including the fermentation reaction resulting in a fermented product. During the fermentation reaction sample collection is effected from which DNA and/or RNA extraction is conducted. Optionally, a bar coding of a sample and the data is conducted. After extraction, sequencing of the DNA and/or RNA is performed. Optionally, the sequencing includes an error correction. Thus, metagenomic markers are obtained. Further, identification and quantification of the molecular pattern based on the DNA and/or RNA sequencing is conducted, for example, based on taxa present or k-mers determined. The data obtained from the DNA and/or RNA sequencing, the metagenomics marker, are included in a database of genomic sequences for taxonomic identification. Comparing the metagenomic marker with the information from the database, it is possible to identify and quantify known taxon's in the samples, thus, a pattern of metagenomic markers of fermented product is obtained. Based on the pattern of metagenomic marker of fermented product, it is possible to compare the pattern with the pattern from a database here identified as a process database for training purposes but also for correlation, namely correlating the comparison of the pattern of the fermented product with the pattern from the database to allow prediction of the fermentation process outcome as well as the further fermentation process and, if necessary, based thereon regulating the ongoing fermentation process.

Further data included are shown in the flowchart on the left side, namely, user information collection. The user information collection falling under the metainformation and reaction parameters according to the present invention include ingredients, reactants, machinery, process (incubation, cycles and steps), culinary/dietary preferences, desired process specificities, desired health effect from fermented product including probiotics, geo-climatic information and time and date. Some of the information are provided by a database including geographical and climate information. The information represents a vector of the fermentation parameters which are compared together with the metagenomics marker information in the process database, thus, allowing correlation according to the present invention.

On the right side, additional parameters are identified including the reaction parameters analyzed in combination with the metagenomics marker information. The reaction parameter includes process related information including recorded temperature cycles but also physico chemical analyses including pH and viscosity as well as the assessment of culinary/dietary qualities. The data obtained represents on the one hand quality measures of the sample e.g. probiotic effect and toxicity which are analyzed in a database optionally together with the identification and quantification of known taxa in the sample. Thus, allowing information on the taxa present in the fermented products and all the quality of the fermented product. In addition, these reaction parameters representing quality measures of the fermented product are also combined with the metagenomics marker pattern to allow comparison with the information present in the process database.

The skilled person is well aware of suitable database and algorithms allowing the analysis of the data. For example, taxonomic identification of reads is performed by mapping of reads against databases of microbial genomes in identification of best matches e.g. using algorithms such as Kraken. The k-mer characterization and counts from sequencing reads is performed after taxonomic identification using algorithms such as Jellyfish.

Examples of parameter and quality measures for different purposes are provided below:
**for silage feed:** parameters include harvested crop species and/or varieties, location of crop growing, crop maturity at harvest, climatic conditions before, during and after harvest, size of chopped silage, target animal or biogas use, location of ensilage, ensilage duration, ensilage storage methods, type of inoculant used, additives used; quality measures include silage spoilage by plant, viral, bacterial or fungal contamination, probiotic potential of silage, % dry matter loss during ensiling, digestibility, pH, concentration of lactic acid, acetic acid, butyric acid, 1,2-propanadiol, propionic acid, amines (cadaverine, glucosamine, histamine, putrescine and tyramine), ammoniac, CO2, nitrate, nitrite, oxide gases of nitrogen, fungal toxins (DON, zearolone, aflatoxin), health impacts on humans and/or animals from exposure or ingestion of silage, impact of silage on dairy production or animal performance, impact of silage on quality of dairy products and processes for making dairy products; interventions include use of additives such as yeast, acetic acid, active charcoal.
**for yoghurt or other fermented dairy products:** parameters include location of fermentation, temperature cycles used, type of input milk, type of inoculant used, type of additives used, machinery used, duration of fermentation, storage methods; quality measures include fermented product spoilage by viral, bacterial or fungal contamination, probiotic potential, digestibility, palatability, pH, acidity, viscosity, protein content, visual appearance, health impacts on humans from exposure or ingestion of fermented dairy product, impact of fermented product on quality of products derived from it.

The present invention is described by way of example without limiting the same. The skilled person is well aware of embodiments falling within the scope of the present invention.

## Claims

1. A method for monitoring fermentation processes comprising the steps of
a) obtaining a sample from the fermentation process;
b) determining a pattern including metagenomic marker;
c) comparing the pattern including metagenomic marker with pattern comprising metagenomic marker from a database;
d) correlating the comparison in step c) with the ongoing fermentation process and predicting the fermentation process outcome.

2. A method for predicting fermentation processes comprising the steps of
a) obtaining a sample from the fermentation process;
b) determining a pattern comprising metagenomic marker,
c) comparing the pattern comprising metagenomic marker with pattern comprising metagenomic marker from a database;
d) predicting the further fermentation process, in particular, the outcome of the fermentation process based on the comparison in step c).

3. A method for regulating fermentation processes comprising the steps of
a) obtaining a sample from the fermentation process;
b) determining a pattern comprising metagenomic marker;
c) comparing the pattern comprising metagenomic marker with pattern comprising metagenomic marker from a database;
d) regulating the ongoing fermentation process based on the comparison in step c).

4. A method according to any one of the preceding claims wherein the fermentation process is a biological fermentation process.

5. The method according to any one of the preceding claims wherein the metagenomic marker include DNA/RNA pattern, in particular, wherein the metagenomic data include data on the microbiome present in said sample.

6. The method according to any one of the preceding claims wherein the determination of the pattern comprising metagenomic marker is a quantification of said metagenomic marker.

7. The method according to any one of the preceding claims further comprising determining additional metainformation before or at the time point when obtaining the sample from the fermentation process and comparing pattern comprising metagenomic marker and metainformation of said sample with the pattern comprising metagenomic marker and metainformation from a database, in particular, wherein said metainformation include date or time and geolocation of the fermentation process as well as past, current and predicted future weather information for the specific location of said fermentation process.

8. The method according to any one of the preceding claims determining reaction parameters of the fermentation reaction obtained before or at the time point when obtaining sample from the fermentation process according to step a), comparing said reaction parameters in combination with the pattern comprising metagenomic marker and, optionally, metainformation, with reaction parameters in combination with the pattern comprising metagenomic marker and, optionally, metainformation, from a database to predict or regulate the future fermentation process, in particular, wherein the reaction parameters include batch number, operators, ingredients and reactants, ferments, machinery, their quantities and/or properties; process related information including recorded temperature cycles; physico- chemical analysis including pH, viscosity, assessment of culinary/dietary qualities using which further data may be retrieved from database such as chemical composition of an intrant, brand identification of an intrant derived from a description or a barcode, predicted health impact of fermented product used.

9. The method according to any one of the preceding claims wherein prediction include the quality of the fermentation product and/or the future pattern of metagenomic markers of the fermented product.

10. The method according to any one of the preceding claims further comprising corrective interventions to improve the future quality of the fermented product based on known impacts of interventions on quality of fermented products from a database.

11. The method according to any one of the preceding claims wherein the fermentation process is a fermentation process for the production of food, feed or bio-energy.

12. The method according to any one of the preceding claims wherein the comparing step c) and the correlating, predicting or regulating step d) is effected by software.

13. Computer program product residing on a computer readable medium, with a program code when it is running on a computer or is loaded on a computer, causes the computer to perform a method according to any one of claims 1 to 12.

14. A computer program storage medium which comprise a computer program product according to claim 13.

15. Apparatus comprising a computer readable storage medium containing program instructions for carrying out the method according to any one of claims 1 to 12 when it is running on a computer.

16. System for at least one of monitoring a fermentation process, predicting a fermentation process, or regulating a fermentation process comprising a device for obtaining a pattern comprising metagenomic marker and a data processing unit comprising a software or a computer program product according to claim 13 for carrying out the method according to any one of claims 1 to 12.
